(19) Europäisches Patentamt
European Patent Office
Office européen des brevets

(11) **EP 1 872 725 A1**

(12) **EUROPEAN PATENT APPLICATION**
published in accordance with Art. 153(4) EPC

(43) Date of publication:
**02.01.2008 Bulletin 2008/01**

(51) Int Cl.:
***A61B 10/00*** (2006.01)     ***G01N 21/35*** (2006.01)

(21) Application number: **06745563.4**

(22) Date of filing: **21.04.2006**

(86) International application number:
**PCT/JP2006/308432**

(87) International publication number:
**WO 2006/115207 (02.11.2006 Gazette 2006/44)**

(84) Designated Contracting States:
**DE FR**

(30) Priority: **22.04.2005 JP 2005125579**
**28.11.2005 JP 2005342598**

(71) Applicant: **Kanazawa University**
**Kanazawa-shi,**
**Ishikawa 920-1192 (JP)**

(72) Inventors:
• **TANAKA, Shigeo,**
**c/o Kanazawa University**
**-shi Ishikawa 9201192 (JP)**

• **NOGAWA, Masamichi,**
**c/o Kanazawa University**
**Kanazawa-shi Ishikawa 9201192 (JP)**
• **YAMAKOSHI, Kenichi,**
**c/o Kanazawa University**
**Kanazawa-shi Ishikawa 9201192 (JP)**

(74) Representative: **Feldmeier, Jürgen et al**
**Prüfer & Partner GbR**
**Patentanwälte**
**Sohnckestraße 12**
**D-81479 München (DE)**

(54) **BONE DENSITY MEASURING DEVICE**

(57)    A small and inexpensive, noninvasive bone density measuring device is provided. A measuring part of the bone density measuring device is constituted by a light emitter 120, which emits near-infrared light, and a light receiver 130, which receives light via a bone of a measuring subject, arranged in a holder 110. Bone density is measured by inserting an arm, for example, in the holder 110 and measuring light absorption (absorbance) by the arm bone. The light emitter 120 and the light receiver 130 are connected to a control unit 140. The control unit 140 controls the light emitter 120 to emit light, inputs a measured value from the light receiver 130, and displays it as bone density. In order to remove the influence of light from the background or difference in bone thickness, ratio of absorbance between two wavelengths is preferably employed. In order to obtain light of two wavelengths, use of a single light receiving element is possible by making two light emitting elements (LEDs) alternately emit light even in the case of using two light emitting elements.

FIG.1

**Description**

**Technical Field**

The present invention relates to a bone density measuring device, which measures bone density using light.

**Background Art**

Currently, the number of osteoporosis victims in Japan is said to be approximately 10 million, and osteoporosis is a serious problem for the future of the aging society. Since lifestyle habits can be a major contributor to osteoporosis, it is necessary to measure bone density on a regular basis to know the state of the bones. Most of the currently used bone density measuring devices utilize X-rays and ultrasound and are thus large and expensive. Therefore, it is difficult for individuals to self-check bone density on a daily basis.

**DISCLOSURE OF THE INVENTION**

[Problem to be Solved by the Invention]

An objective of the present invention is to provide a small and inexpensive, noninvasive bone density measuring device allowing individuals to measure bone density daily.

[Means of Solving the Problem]

In order to achieve the above-mentioned objective, the present invention includes: a light emitter, which emits light of at least two wavelengths; a light receiver, which receives light from the light emitter via a bone; and a control unit, which is connected to the light emitter and the light receiver to control the light emitter, input a signal from the light receiver, and display it as bone density from absorbance of light of multiple wavelengths.

The light emitter may emit light of two wavelengths, and the control unit may display bone density represented by ratio of absorbance or difference in absorbance of light of the two wavelengths.

The light emitter preferably emits near-infrared light of two wavelengths $\lambda_1$ and $\lambda_2$ with which change in absorbance becomes greater as bone density changes.

The light emitter should provide a combination of the two wavelengths ($\lambda_1$ and $\lambda_2$) such that correlation coefficient (r) between bone density and ratio of absorbance or difference in absorbance is 0.99 or greater and slope (s) is 10,000 or less, and emits light of a wavelength region of near-infrared LEDs used as the light emitter in which absorption by skin, water, and fat is minimum.

The control unit should drive two light-emitting elements of the light emitter alternatively to emit light of two wavelengths, and the light receiver should be controlled so as to time-division multiplex and receive light of the two wavelengths by a single light receiving element.

The light emitter should drive multiple light emitting elements sequentially to emit light of multiple wavelengths, and the light receiver should receive light of the multiple wavelengths by a single light receiving element.

The light emitter and the light receiver should be deployed so as to receive transmitted light and reflected and scattered light via the bone.

[Effects of Invention]

According to the present invention, a small and inexpensive, noninvasive bone density measuring device can be provided by measuring light absorption by bone, as described above. This allows daily measurement of bone density by individuals.

**BRIEF DESCRIPTION OF DRAWINGS**

FIG. 1 is a diagram showing a general structure of a bone density measuring device according to the present invention;
FIG. 2 is a diagram showing a light absorption spectrum by a bone;
FIG. 3-1 is a diagram showing correlation between ratio of absorbance of two wavelengths and bone density;
FIG. 3-2 is another diagram showing correlation between ratio of absorbance of two wavelengths and bone density;
FIG. 3-3 is a diagram showing correlation between difference of absorbance of two wavelengths and bone density;

FIG. 4(a) is a graph showing a near-infrared region absorbance spectrum for a bone; FIG. 4(b) is cross-sectional pictures of a bone showing bone density;

FIG. 5(a) is a graph showing a relationship between ratio of absorbance of two wavelengths and bone density and a relationship between difference in absorbance of the two wavelengths and bone density; FIG. 5(b) is a diagram showing distribution of slope (s) and correlation coefficient (r); FIG. 5(c) is a partially enlarged view of the distribution of slope (s) and correlation coefficient (r);

FIG. 6-1 is a diagram showing combinations of two wavelengths ($\lambda_1$, $\lambda_2$) where correlation coefficient (r) of the ratio of absorbance is 0.99 or greater and slope (s) is 10,000 or less;

FIG. 6-2 is a diagram showing combinations of two wavelengths ($\lambda_1$, $\lambda_2$) where correlation coefficients (r) of ratio of absorbance (a) and difference in absorbance (b) are 0.99 or greater and slope (s) is 10,000 or less;

FIG. 7 is a diagram showing an absorbance spectrum of skin;

FIG. 8 is a diagram showing absorbance spectrums of water and fat;

FIG. 9-1 is an enlarged diagram of a region (framed region) including combinations of two wavelengths ($\lambda_1$, $\lambda_2$) selected from a region C shown in FIG. 6-1 for reducing influences of water and fat within the skin and body;

FIG. 9-2 is an enlarged diagram of a region (framed region) including combinations of two wavelengths ($\lambda_1$, $\lambda_2$) selected from regions C and F shown in FIG. 6-2 for reducing influences of water and fat within the skin and body;

FIG. 10(a) is a schematic showing how to measure for a protrusion of the distal ulna; FIG. 10(b) is an X-ray of the protrusion of the distal ulna;

FIG. 11 shows a measuring unit for measuring bone density: (a) is a picture showing a side; and FIG. 11(b) is picture showing the front of the measuring unit;

FIG. 12(a) is a time chart showing light emitting timings of a light emitter (two LEDs) ; FIG. 12(b) is graph giving measuring timings of a light receiver (PD);

FIG. 13-1 shows relationships between ratio of absorbance and density of artificial bones: (a) $\lambda_1$: 850 nm, $\lambda_2$: 1550 nm; (b) $\lambda_1$: 1050 nm, $\lambda_2$: 1550 nm; (c) $\lambda_1$: 1200 nm, $\lambda_2$: 1550 nm;

FIG. 13-2 shows relationships between ratio of absorbance, difference in absorbance, and density of artificial bones (background excluded): (a) $\lambda_1$: 850 nm, $\lambda_2$: 1550 nm; (b) $\lambda_1$: 1050 nm, $\lambda_2$: 1550 nm; (c) $\lambda_1$: 1200 nm, $\lambda_2$: 1550 nm;

FIG. 14 shows a relationship between absorbance and density of an artificial bone; and

FIG. 15 is a schematic showing transmission, reflection, and dispersion of light in measuring of the artificial bone: (a) shows the case of low bone density; and (b) shows the case of high bone density.

## BEST MODE FOR CARRYING OUT THE INVENTION

The present invention constitutes a bone density measuring device using near-infrared light excellent in biological permeability. The bone density measuring device of the present invention is described while referring to the appended drawings.

A bone tissue is constituted by a bone and bone marrow surrounding the bone. 'Bone' in this case means a bone matrix having hydroxyapatite and collagen tissue as main components. Furthermore, bone density means space occupancy or porosity of the 'bone' indicated by weight per unit space, and the bone density measuring device described forthwith is what measures this bone density.

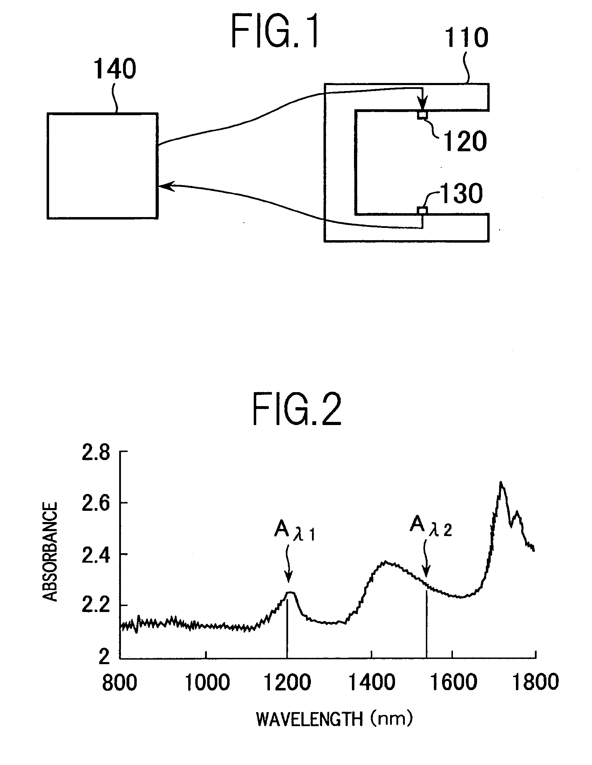

FIG. 1 is a schematic showing a general structure of the bone density measuring device of the present invention. In FIG. 1, a measuring part of the bone density measuring device is constituted by a light emitter 120, which emits near-infrared light, and a light receiver 130, which receives light via a bone of a measuring subject, arranged in a holder 110. Bone density is measured by inserting an arm, for example, in the holder 110 and measuring light absorption (absorbance) by the armbone. The light emitter 120 and the light receiver 130 are connected to a control unit 140. The control unit 140 controls the light emitter 120 to emit light, inputs a measured value from the light receiver 130, and displays it as bone density.

A light emitting diode (LED), for example, may be used as the light emitter 120, and a photo diode, for example, may be used as a light receiving element for receiving near-infrared light from the light emitter 120.

The holder 110 should have an adjustable distance between the light emitter 120 and the light receiver 130. Furthermore, the holder 110 may be a watch band type, for example.

In order to remove the influence of light from the background or influence of difference in bone thickness, use of ratio of absorbance between two wavelengths is preferred. Difference in absorbance may also be used.

In this case, the light receiver must differentiate the two wavelengths and then receive light. Therefore, usually, two light receiving elements capable of selective detection using wavelength filters are necessary. However, a structure with two light emitting elements (LEDs) emitting different wavelengths alternately may allow use of a single light receiving element.

An example where a cancellous bone specimen including bone marrow cut out from a distal end of a bovine femur (knee joint) is measured is given below. FIG. 2 shows a near-infrared region light absorption spectrum of the bone specimen. In the spectrum, peaks are seen near 1200 nm and 1460 nm, which is the absorption wavelength of water.

[Working Example 1]

Two optimum near-infrared wavelengths must be selected for measuring. The selected wavelengths in the measuring example below are 1200 nm ($\lambda_1$) and 1540 nm ($\lambda_2$). The vicinity of wavelengths where water absorption is great is avoided.

Absorbance A is defined as A=(log($I_0$/I))/L, where $I_0$ denotes incident light intensity, I denotes transmitted light intensity, and L denotes specimen thickness.

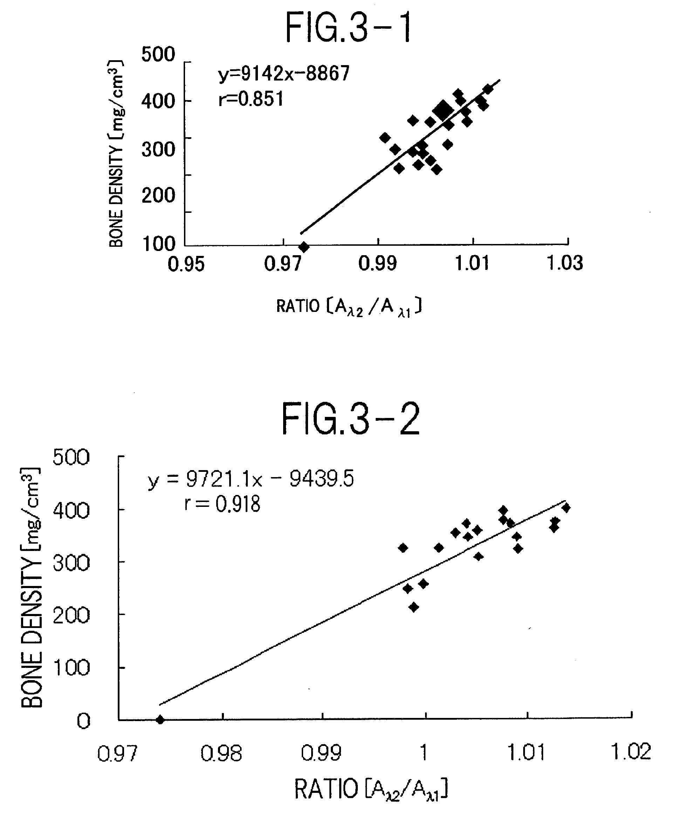

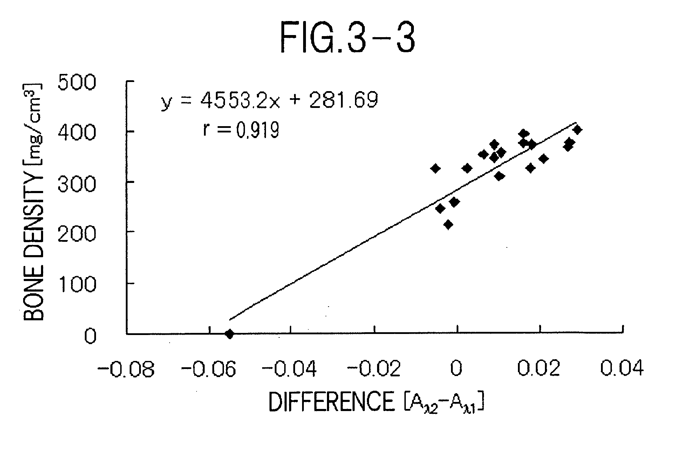

The measuring example of ratio of absorbance (A$\lambda_1$/A$\lambda_2$) for the two wavelengths is given in FIG. 3-1. Note that the ratio of absorbance for bone density 0 is calculated based on a bone marrow absorbance spectrum. FIG. 3-1 shows a positive correlation (correlation coefficient r=0.851), where bone density can be measured from the ratio of absorbance of the two wavelengths.

<Other Measuring Examples>

FIGS. 3-2 and 3-3 show measuring examples of ratio of absorbance of the two respective wavelengths (FIG. 3-2) and difference (FIG. 3-3) thereof except for artificially adjusted bone density of the samples used for data analysis of FIG. 3-1. Bovine cancellous bone has higher bone density than human cancellous bone and thus it is very difficult to obtain human cancellous bone density (particularly density of an osteoporosis level) frombovines. Therefore, in FIG. 3-1, samples with small bone density are prepared by shaving a cancellous trabecular bone using a knife, and then measured. However, since samples prepared by a person in this way lose light scattering characteristics intrinsic to genuine trabecular bone structure, different tendencies than with unprocessed samples are seen. Therefore, data for these samples is omitted, thereby providing better correlation. The graphs given in FIGS. 3-2 and 3-3 show positive correlations (ratio of absorbancer=0.918, difference in absorbance r=0.919). These indicate that bone density can be measured from ratio of absorbance and difference in absorbance.

<Selection of Two Wavelengths>

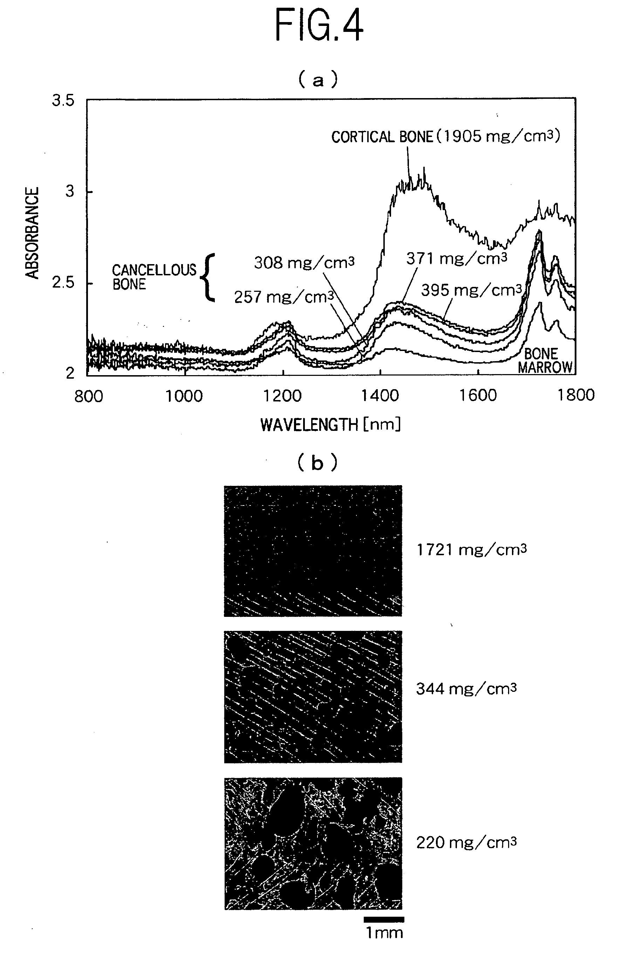

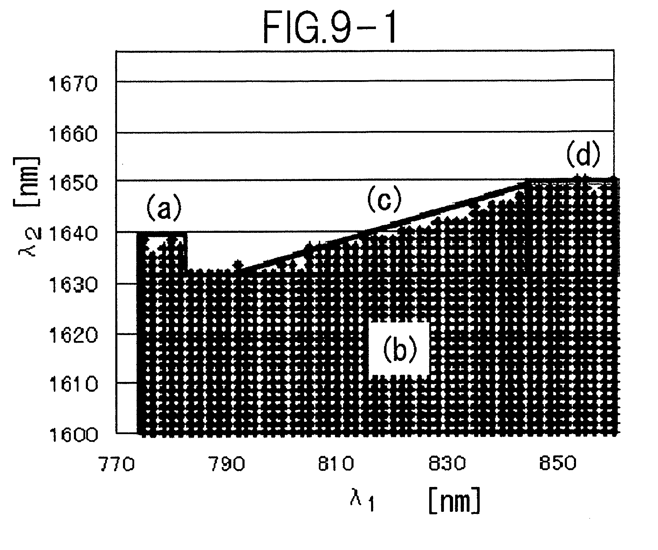

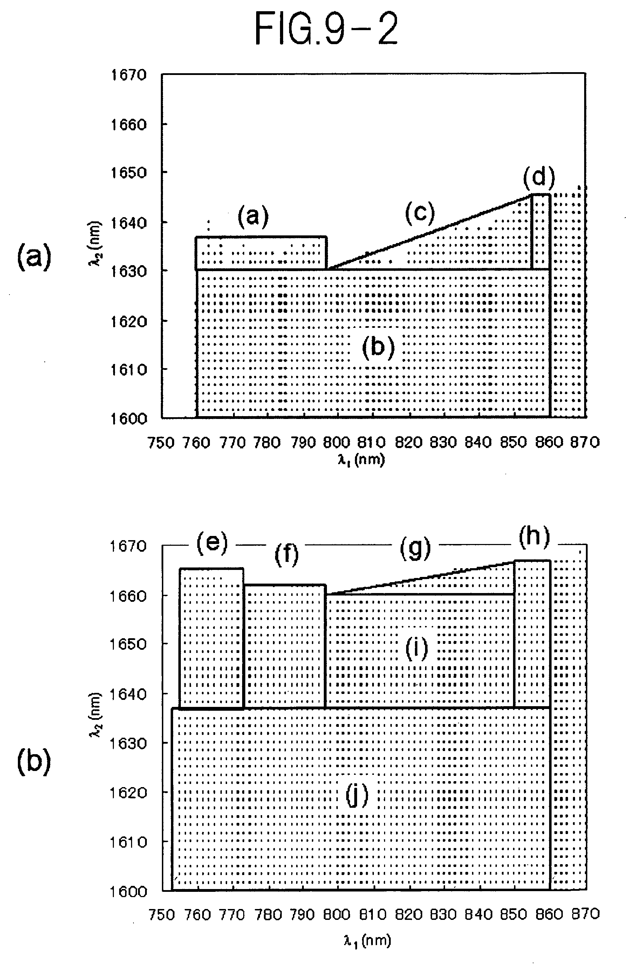

In the above-given example, two wavelengths outside of the vicinity of wavelengths where absorbance of water is great are used; however, two optimal wavelengths for the measuring are selected by analyzing near-infrared light for the bone samples taken from the bovine femur. This is described forthwith using FIGS. 4 through 9-2.

FIG. 4(a) shows a near-infrared region absorbance spectrum for bones with various bone densities (only bone marrow: 0 mg/cm$^3$, cancellous bones: 257 mg/cm$^3$, 308 mg/cm$^3$, 371 mg/cm$^3$, 395 mg/cm$^3$, and cortical bone: 1905 mg/cm$^3$). FIG. 4(b) shows pictures of cross sections of bones with various densities (1721 mg/cm$^3$, 344 mg/cm$^3$, 220 mg/cm$^3$). As shown in FIG. 4(a), the absorbance spectrum shows peaks near 1200 nm, 1450 nm, and 1750 nm.

With the two near-infrared light wavelengths, bone density should be measured utilizing the fact that the greater bone density changes, the greater absorbance changes. This is because, when considering transmission of light in a bone tissue, there is little increase in absorbance with the wavelength $\lambda_1$ due to increase in bone density whereas there is great increase in absorbance with the wavelength $\lambda_2$, resulting in ratio of absorbance and difference in absorbance defined by the following equations having a positive correlation with bone density. Selection of two wavelengths with such largely different changes in absorbance due to such changes in bone density allows provision of a further sensitively structured bone density measuring device.

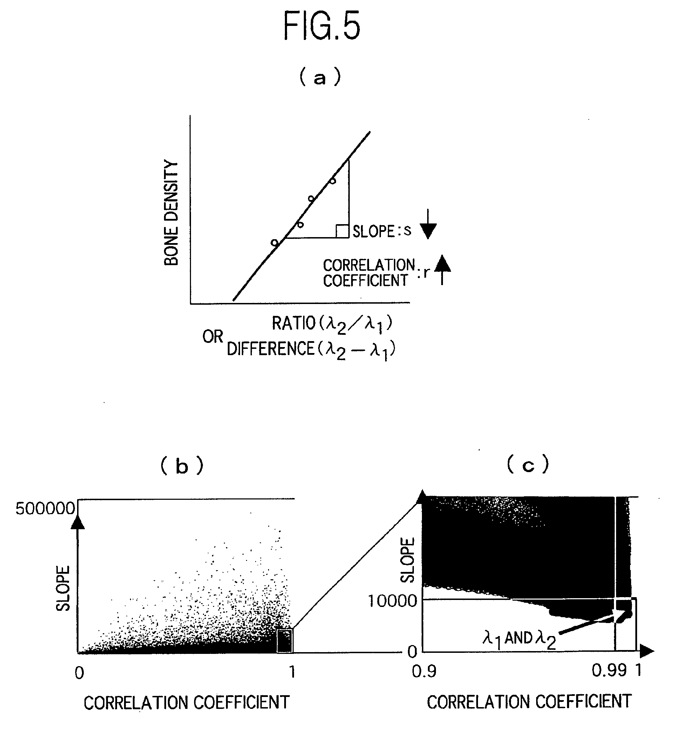

Data of absorbance of near-infrared light wavelengths and various bone densities as shown in FIG. 4(a) is used to select two wavelengths with largely different changes in absorbance due to changes in bone density. As a selection method, for example, a method of examining all relationships between bone densities and corresponding respective ratios of absorbance of near-infrared light of the two wavelengths or between the bone densities and corresponding respective differences in absorbance thereof, as shown in FIG. 5(a). Ratio of absorbance of the two near-infrared lights and difference in absorbance thereof are calculated in the following manner.

$$\text{Ratio of absorbance} = \log/[I_0/I]_{\lambda 2} \; / \; \log/[I_0/I]_{\lambda 1} \; = \; \mu_{\lambda 2}L / \mu_{\lambda 1}L$$

$$\text{Difference in absorbance} = \log/[I_0/I]_{\lambda 2} \; - \; \log/[I_0/I]_{\lambda 1} \; = \; (\mu_{\lambda 2} - \mu_{\lambda 1})L$$

In these equations, $\mu_{\lambda 1}$, $\mu_{\lambda 2}$ are attenuation coefficients for the wavelengths $\lambda_1$ and $\lambda_2$ and parameters dependant on bone density, where L denotes light path length, $I_0$ denotes incident light intensity, and I denotes output light intensity. Note that the attenuation coefficients include both attenuation due to light absorption and attenuation due to light scattering. The above-given equations give either ratio of absorbance or difference in absorbance when the two wavelengths have the same light path length.

Numerical values obtained from the relationship between bone density and ratio of absorbance or relationship between the bone density and difference in absorbance are slope s and correlation coefficient r when collinear approximation of the relationship is carried out, as shown in FIG. 5(a). Exemplary distributions of the resulting slopes s and correlation coefficients r are shown in FIGS. 5(b) and 5(c). As shown in FIG. 5(a), the smaller the slope s, the greater the resolution of the bone density; where the horizontal axis represents ratio of absorbance or difference in absorbance while the vertical axis represents bonedensity. FIG. 5(c) is an enlarged region of the distribution diagram of FIG. 5(b) with a slope of 30000 or less and correlation coefficient of 0.9 or greater.

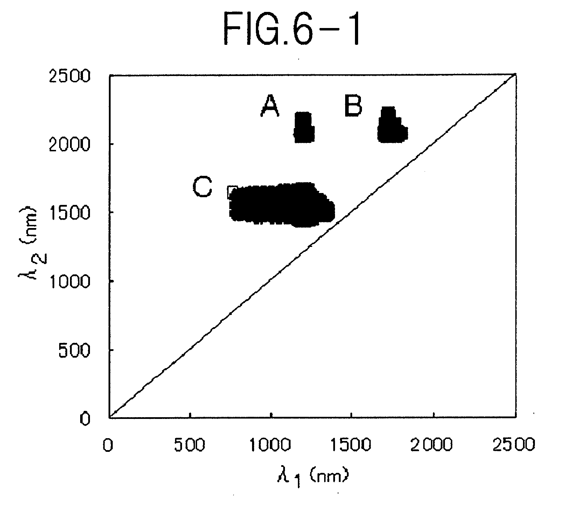

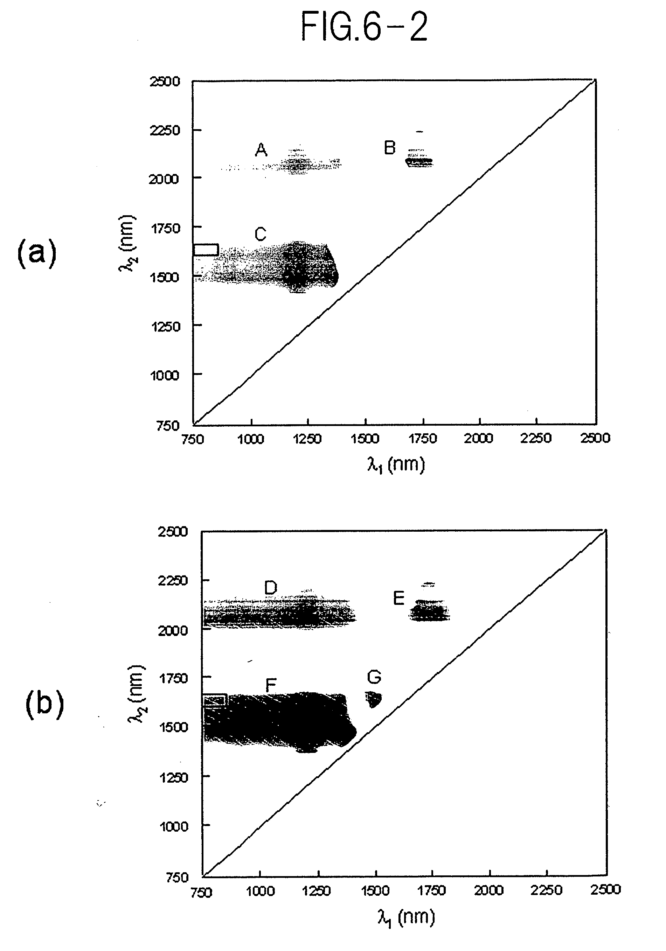

Here, FIG. 6-1 shows distribution of sets of two wavelength near-infrared lights, which have small slopes and strong correlation in the case of ratio of absorbance, for example, a slope of 10,000 or less and correlation coefficient of 0.99 or greater; where the horizontal axis represents $\lambda_1$ while the vertical axis represents $\lambda_2$. The two wavelengths as shown in FIG. 6-1 are distributed in three regions A, B, and C. Note that the ranges of the regions given below are maximum and minimum wavelengths for each region.

Region A: $\lambda_1$:1168 nm to 1243 nm, $\lambda_2$:2000 nm to 2193 nm
Region B: $\lambda_1$:1686 nm to 1806 nm, $\lambda_2$:2030 nm to 2220 nm
Region C: $\lambda_1$:775 nm to 1373 nm, $\lambda_2$:1416 nm to 1676 nm

The two wavelengths belonging to region C should be employed taking into account the wavelength region (850 nm to 1550 nm) which most of the commercially available near-infrared LEDs output.

FIG. 6-2 shows measuring examples of ratio of absorbance (FIG. 6-2(a)) of the two wavelengths and difference in absorbance (FIG. 6-2(b)) thereof except for artificially adjusted bone density of the samples used for data analysis of FIG. 6-1.

In the case of ratio of absorbance as shown in FIG. 6-2(a), the two wavelengths are mainly distributed in three regions A, B, and C. On the other hand, in the case of difference in absorbance as shown in FIG. 6-2(b), the two wavelengths are mainly distributed in four regions D, E, F, and G. Note that the ranges of the regions given below are maximum and minimum wavelengths for each region.

Region A: $\lambda_1$:755 nm to 1410 nm, $\lambda_2$:2001 nm to 2185 nm
Region B: $\lambda_1$:1666 nm to 1809 nm, $\lambda_2$:2038 nm to 2232 nm
Region C: $\lambda_1$:755 nm to 1383 nm, $\lambda_2$:1403 nm to 1675 nm
Region D: $\lambda_1$:751 nm to 1440 nm, $\lambda_2$:1988 nm to 2219 nm
Region E: $\lambda_1$:1618 nm to 1837 nm, $\lambda_2$:2016 nm to 2239 nm
Region F: $\lambda_1$:751 nm to 1427 nm, $\lambda_2$:1368 nm to 1682 nm
Region G: $\lambda_1$:1453 nm to 1539 nm, $\lambda_2$:1605 nm to 1685 nm

The two wavelengths belonging to regions C, F, and G should be employed taking into consideration the wavelength region (850 nm to 1550 nm) which most of the commercially available near-infrared LEDs output.

<Skin and Absorbance of Fat and Water>

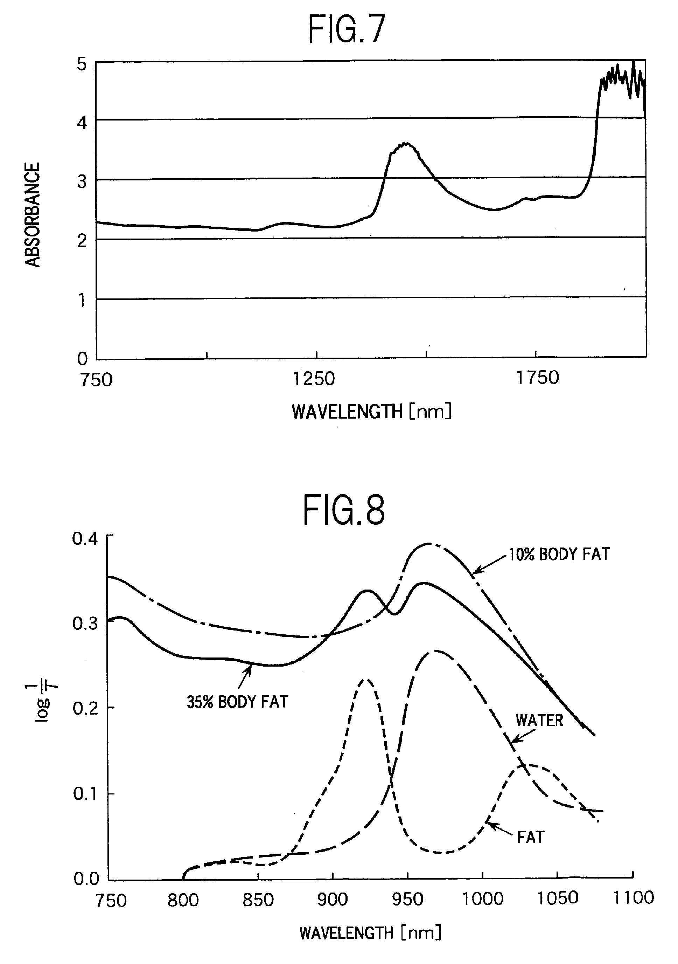

Since near-infrared light is absorbed by skin, water and fat, near-infrared light allowing minimum influences thereof should be selected. FIG. 7 shows an absorption spectrum of skin. In this diagram, peaks are seen near wavelengths 1850 nm to 2200 nm and 1350 nm to 1600 nm. Furthermore, increase is seen near 1100 nm. Accordingly, in order to minimize influence of skin, selection of wavelengths of approximately 1100 nm or less or between approximately 1600 nm and 1850 nm is preferred.

FIG. 8 is a diagram showing absorbance spectrums of water and fat. This graph in FIG. 8 is cited from the following paper. Conway JM, Norris KH, Bodwell CE. : "A new approach for the estimation of body composition: infrared interactance "The American Journal of Clinical Nutrition, Vol. 40, No. 6, pp.1123-1130, 1984

In this graph, absorbance of near-infrared light shows peaks near 970 nm for water and 930 nm and 1030 nm for fat. According to this drawing, it is preferable to avoid the range of wavelengths between 860 nm and 1100 nm.

FIG. 9-1 is an enlarged diagram of a region (framed region in region C of FIG. 6-1) including combinations of two wavelengths ($\lambda_1$, $\lambda_2$) in region C selected for reducing influences of water and fat within the skin and body.

In order to avoid influences of water and fat within the skin and body, it is desirable to select such a range of two wavelengths as given below, for example, shown in FIG. 9-1 from the two wavelength near-infrared lights belonging to region C.

(a) A rectangular region given by four points with coordinates ($\lambda_1 \approx 775$ nm, $\lambda_2 \approx 1640$ nm) , ($\lambda_1 \approx 780$ nm, $\lambda_2 \approx 1640$ nm), ($\lambda_1 \approx 780$ nm, $\lambda_2 \approx 1630$ nm), and ($\lambda_1 \approx 775$ nm, $\lambda_2 \approx 1630$ nm) (FIG. 9-1(a))
(b) A rectangular region given by four points with coordinates ($\lambda_1 \approx 775$ nm, $\lambda_2 \approx 1630$ nm), ($\lambda_1 \approx 775$ nm, $\lambda_2 \approx 1600$ nm), ($\lambda_1 \approx 860$ nm, $\lambda_2 \approx 1600$ nm), and ($\lambda_1 \approx 860$ nm, $\lambda_2 \approx 1630$ nm) (FIG. 9-1(b))
(c) A right triangular region given by three points with coordinates ($\lambda_1 \approx 790$ nm, $\lambda_2 \approx 1630$ nm), ($\lambda_1 \approx 845$ nm, $\lambda_2 \approx 1650$ nm), and ($\lambda_1 \approx 845$ nm, $\lambda_2 \approx 1630$ nm) (FIG. 9-1(c))
(d) A rectangular region given by four points with coordinates ($\lambda_1 \approx 845$ nm, $\lambda_2 \approx 1650$ nm), ($\lambda_1 \approx 860$ nm, $\lambda_2 \approx 1650$ nm), ($\lambda_1 \approx 860$ nm, $\lambda_2 \approx 1630$ nm), and ($\lambda_1 \approx 845$ nm, $\lambda_2 \approx 1630$ nm) (FIG. 9-1(d))

Similarly, in FIG. 6-2, it is preferable to select a range from approximately 755 nm to 860 nm (ratio of absorbance region C) or a range from 751 nm to 860 nm (difference in absorbance region F) for $\lambda_1$, and a range from approximately 1600 nm to 1676 nm for $\lambda_2$. This is shown in FIG. 9-2, for example.

FIG. 9-2 shows enlarged diagrams of regions (framed regions C and F of FIG. 6-2) indicating a combination of two wavelengths ($\lambda_1$, $\lambda_2$) selected for reducing influences of water and fat within the skin and body in region C of FIG. 6-2.

In the case of using ratio of absorbance of the two wavelengths, in order to avoid influences of water and fat within the skin and body, it is preferable to select such a range of the two wavelengths as given below and shown in a through d of FIG. 9-2(a) from the two wavelength near-infrared lights belonging to region C.

(a) A rectangular region given by four points with coordinates ($\lambda_1 \approx 760$ nm, $\lambda_2 \approx 1630$ nm), ($\lambda_1 \approx 760$ nm, $\lambda_2 \approx 1637$ nm), ($\lambda_1 \approx 797$ nm, $\lambda_2 \approx 1630$ nm), and ($\lambda_1 \approx 797$ nm, $\lambda_2 \approx 1637$ nm)
(b) A rectangular region given by four points with coordinates ($\lambda_1 \approx 760$ nm, $\lambda_2 \approx 1630$ nm), ($\lambda_1 \approx 760$ nm, $\lambda_2 \approx 1600$ nm), ($\lambda_1 \approx 860$ nm, $\lambda_2 \approx 1600$ nm), and ($\lambda_1 \approx 860$ nm, $\lambda_2 \approx 1630$ nm)
(c) A right triangular region given by three points with coordinates ($\lambda \approx 797$ nm, $\lambda_2 \approx 1630$ nm), ($\lambda_1 \approx 855$ nm, $\lambda_2 \approx 1645$ nm), and ($\lambda_1 \approx 855$ nm, $\lambda_2 \approx 1630$ nm)
(d) A rectangular region given by four points with coordinates ($\lambda_1 \approx 855$ nm, $\lambda_2 \approx 1645$ nm), ($\lambda_1 \approx 860$ nm, $\lambda_2 \approx 1645$ nm), ($\lambda_1 \approx 860$ nm, $\lambda_2 \approx 1630$ nm), and ($\lambda_1 \approx 855$ nm, $\lambda_2 \approx 1630$ nm)

In the case of using difference in absorbance of the two wavelengths, in order to avoid influences of water and fat within the skin and body, it is preferable to select a range of the two wavelengths as given below and shown in e through h of FIG. 9-2(b) from the two wavelength near-infrared lights belonging to region F.

(e) A rectangular region given by four points with coordinates ($\lambda_1 \approx 753$ nm, $\lambda_2 \approx 1665$ nm), ($\lambda_1 \approx 773$ nm, $\lambda_2 \approx 1665$ nm), ($\lambda_1 \approx 773$ nm, $\lambda_2 \approx 1637$ nm), and ($\lambda_1 \approx 755$ nm, $\lambda_2 \approx 1637$ nm)
(f) A rectangular region given by four points with coordinates ($\lambda_1 \approx 773$ nm, $\lambda_2 \approx 1662$ nm), ($\lambda_1 \approx 797$ nm, $\lambda_2 \approx 1662$ nm), ($\lambda_1 \approx 797$ nm, $\lambda_2 \approx 1637$ nm), and ($\lambda_1 \approx 773$ nm, $\lambda_2 \approx 1637$ nm)
(g) A right triangular region given by three points with coordinates ($\lambda_1 \approx 797$ nm, $\lambda_2 \approx 1660$ nm), ($\lambda_1 \approx 850$ nm, $\lambda_2 \approx 1667$ nm), and ($\lambda_1 \approx 850$ nm, $\lambda_2 \approx 1660$ nm)
(h) A rectangular region given by four points with coordinates ($\lambda_1 \approx 850$ nm, $\lambda_2 \approx 1667$ nm), ($\lambda_1 \approx 860$ nm, $\lambda_2 \approx 1667$ nm), ($\lambda_1 \approx 860$ nm, $\lambda_2 \approx 1637$ nm), and ($\lambda_1 \approx 850$ nm, $\lambda_2 \approx 1637$ nm)
(i) A rectangular region given by four points with coordinates ($\lambda_1 \approx 797$ nm, $\lambda_2 \approx 1660$ nm), ($\lambda_1 \approx 850$ nm, $\lambda_2 \approx 1660$ nm), ($\lambda_1 \approx 850$ nm, $\lambda_2 \approx 1637$ nm), and ($\lambda_1 \approx 797$ nm, $\lambda_2 \approx 1637$ nm)
(j) A rectangular region given by four points with coordinates ($\lambda_1 \approx 753$ nm, $\lambda_2 \approx 1637$ nm), ($\lambda_1 \approx 860$ nm, $\lambda_2 \approx 1637$ nm), ($\lambda_1 \approx 860$ nm, $\lambda_2 \approx 1600$ nm), and ($\lambda_1 \approx 753$ nm, $\lambda_2 \approx 1600$ nm)

[Working Example 2]

Results of selecting commercially available LEDs emitting light having wavelengths belonging to the above-given ranges, developing a noninvasive bone density measuring device, and measuring using artificial bones with known densities are given forthwith.

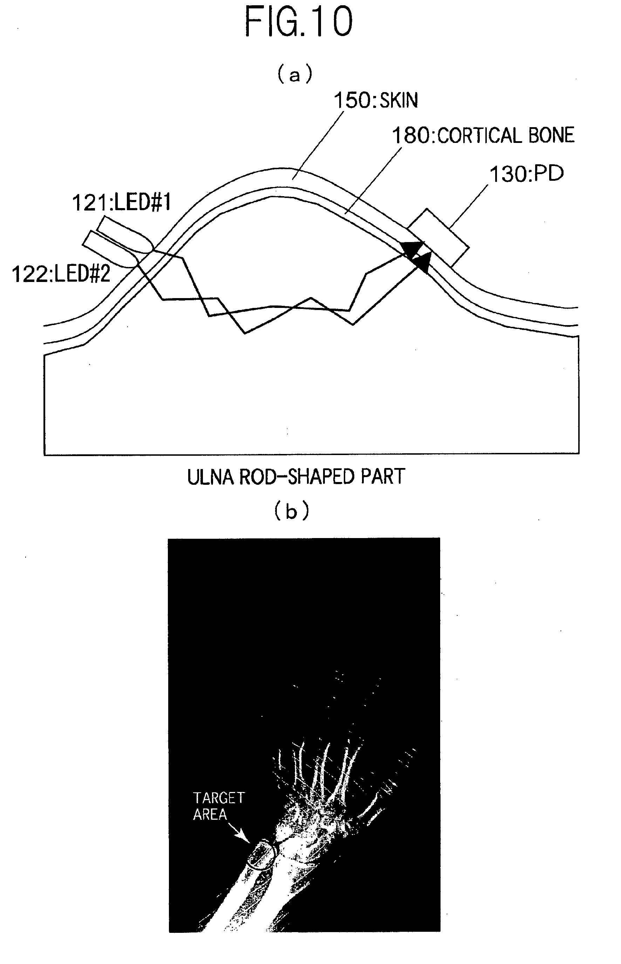

FIG. 10(a) is a schematic diagram showing how to measure a protrusion of the ulna of a wrist, and FIG. 10(b) shows an X-ray of a target area. As shown in FIG. 10(a), a light emitter (two LEDs) and a light receiver (PD) face each other at an angle via the wrist bone. The light emitter emits near-infrared light having two different wavelengths from two LEDs. Light transmitting through the bone and reflecting and scattering is received by the light receiver (PD). Note that the measuring subject may be an ankle since it has the same bone geometry.

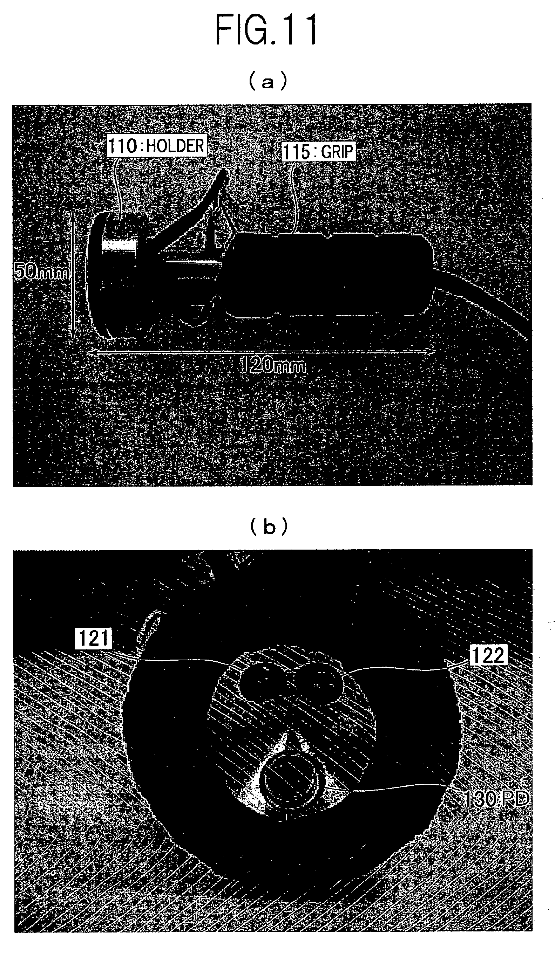

FIG. 11 shows pictures of an actually fabricated measuring unit. FIG. 11(a) is a picture showing a side of the measuring unit, and FIG. 11(b) is a picture showing the front. When the measuring unit in the pictures of FIG. 11 is placed on the wrist bone or measuring subject, a black sponge for blocking light from the outside is attached around the round holder 110 to which LEDs and a PD are secured. As shown in FIG. 11(b), two LEDs 121 and 122 and a PD 130 are provided in a concave portion of the holder 110. When measuring, a grip 115 is grasped, and the unit is pressed against the wrist bone or measuring subj ect tomeasure.

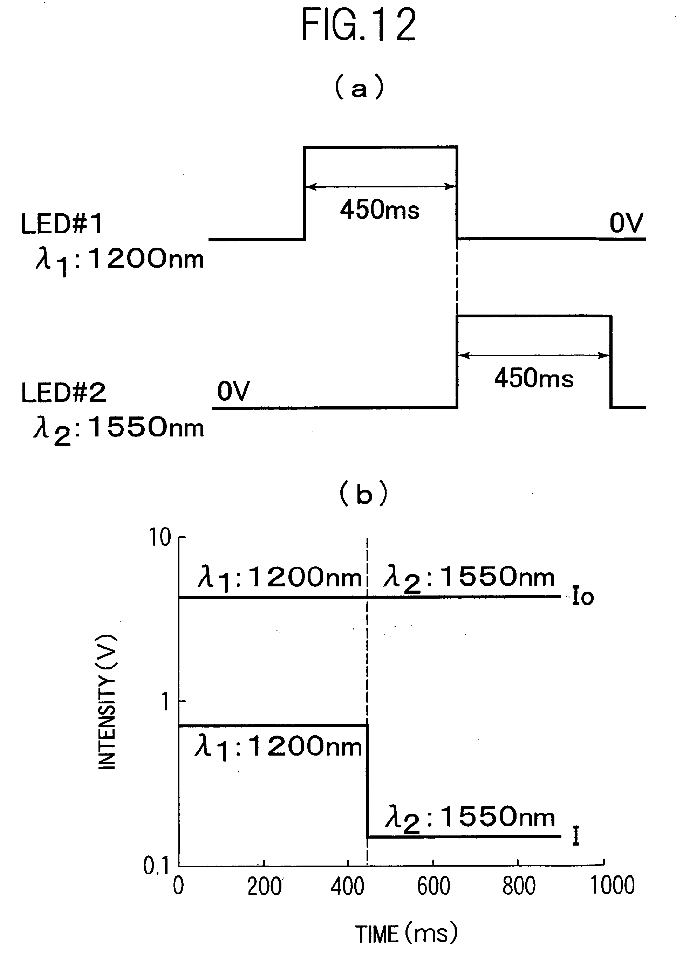

FIG. 12(a) is a time chart showing light emitting timings of the light emitter (two LEDs). FIG. 12(b) is graph showing measuring timings of the light receiver (PD).

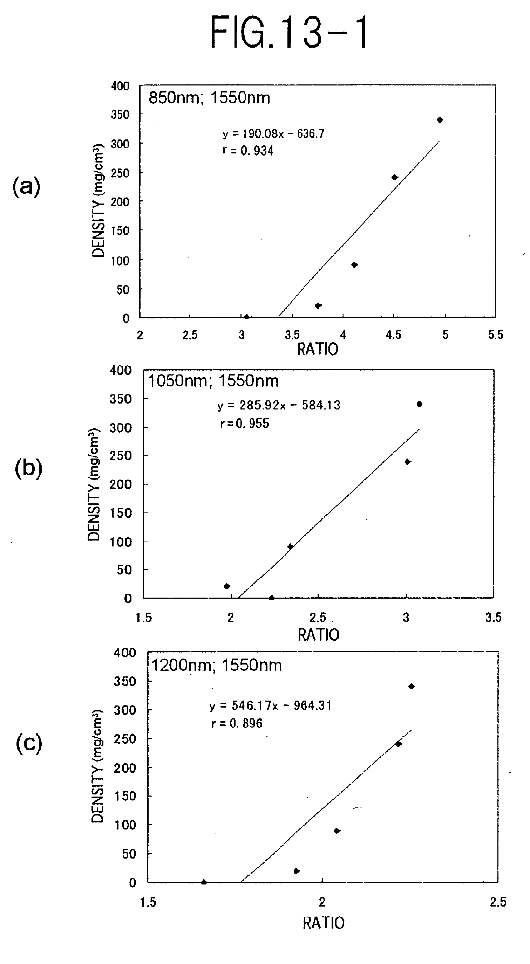

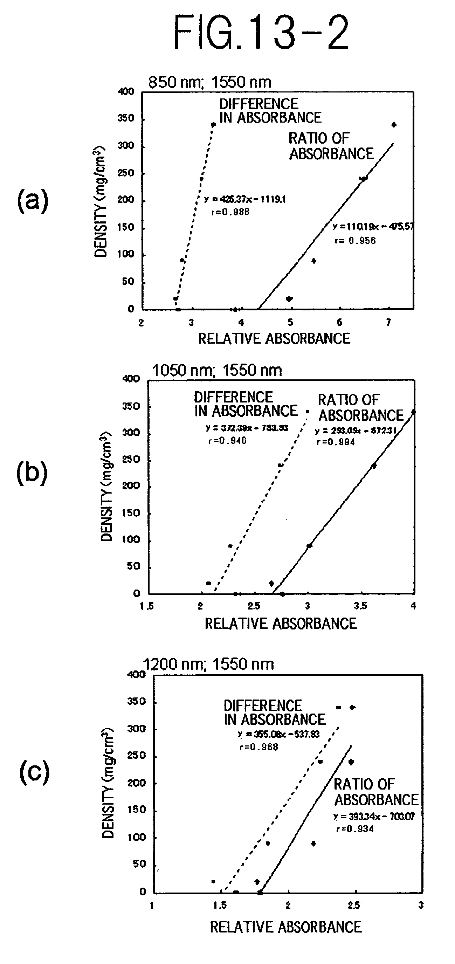

As indicated by the time chart of FIG. 12(a), the light emitter makes two LEDs emit light alternately and a single photo diode (PD) constituting the light receiver receives the light. As shown in FIG. 12(b), wavelength of light being received is identified by adapting the light receiving period of the light-receiving photo diode to the light emitting timings. A control unit not shown in the drawings calculates a ratio of intensity $I_0$ to that of directly received and pre-measured light so as to estimate bone density. Results thereof are given in FIGS. 13-1 and 13-2.

Note that while a case where light emission and light reception are conducted only once with a pulse width of 450 ms is given in FIG. 12, a more reliable measured value may be obtained by repeating light emission and light reception multiple times with a shorter pulse width and then taking the average thereof. Furthermore, this allows a shorter measurement time.

Measurements shown in FIGS. 13-1 (ratio of absorbance) and 13-2 (difference in absorbance) are taken using artificial bone tissues made by mixing gelatin with cancellous bone chips taken from bovine femur. Gelatin and cancellous bone chips are mixed such that spatial densities of the prepared artificial bone tissues are 20, 90, 240, and 340 mg/cm$^3$. The fabricated artificial bone samples are covered with 6 mm of gelatin, forming an artificial skin layer. The shapes are also made the same as the protrusion of the ulna of the wrist.

The wavelengths used for measuring are $\lambda_1$:850 nm, $\lambda_2$:1550 nm in FIGS. 13-1(a) and 13-2(a), $\lambda_1$:1050 nm, $\lambda_2$:1550 nm in FIGS. 13-1(b) and 13-2(b), and $\lambda_1$:1200 nm, $\lambda_2$:1550 nm in FIGS. 13-1(c) and 13-2(c).

Note that FIG. 13-2 takes background (value input to the light receiver when light is not emit from the light emitter) into account when measuring difference in absorbance and ratio of absorbance.

Since the slope of the combinations of the wavelengths ($\lambda_1$:850 nm, $\lambda_2$:1550 nm) of FIG. 13-1(a) is the least in the ratio of absorbance shown in FIG. 13-1, the bone density may be predicted more sensitively. Furthermore, the correlation coefficient is most favorable for the combination of the wavelengths ($\lambda_1$:1050 nm, $\lambda_2$:1550 nm) of FIG. 13-1(b).

Meanwhile, in the case of difference in absorbance, since the slope of the combinations of the wavelengths ($\lambda_1$:1200 nm, $\lambda_2$:1550 nm) in FIG. 13-2(c) is the least, the bone density may be predicted more sensitively. Furthermore, the correlation coefficient is most favorable for the combination of the wavelengths ($\lambda_1$:850 nm, $\lambda_2$:1550 nm) of FIG. 13-2(a).

In analysis of the aforementioned near-infrared region absorbance spectrum for bones, when either the ratio of absorbance of the two wavelengths or the difference in absorbance thereof has high correlation with the bone density, linear slopes indicating both relationships are approximately 4500 to 10,000. Meanwhile, in the experiment using the artificial bones, the linear slopes are several hundred, which is very low, as shown in FIGS. 13-1 and 13-2. This indicates that bone density may be more sensitively predicted from the ratio of absorbance or the difference in absorbance with this measurement system.

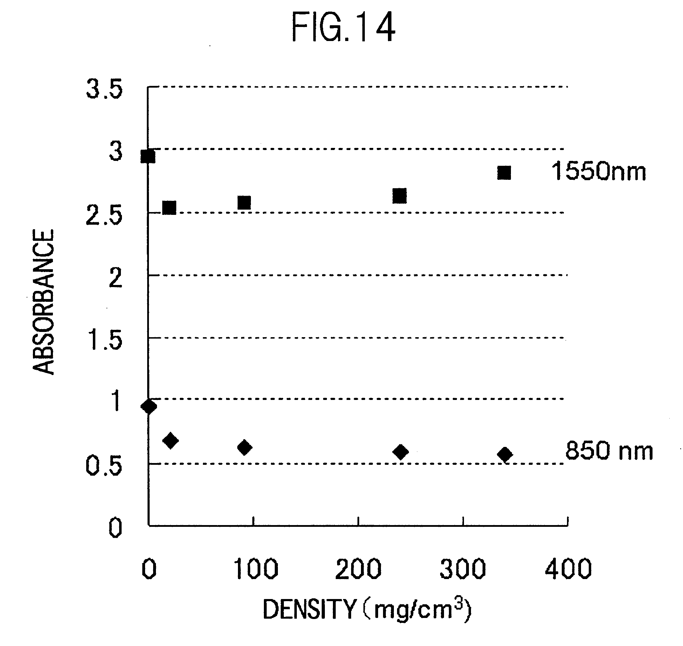

FIG. 14 shows the relationship between absorbance when using the two wavelengths 850 nm and 1550 nm and densities of the artificial bone tissues. It is understood from FIG. 14 that absorbance at 1550 nm has a tendency to increase as density increases, and absorbance at 850 nm has a tendency to decrease. This is a contributing factor to further reduce the linear slope indicating the relationship between density and difference in absorbance.

Results of the working example with the artificial bone tissues may be explained with the following mechanism. In this working example, as shown in the schematic diagram of FIG. 10(a), since the bone is irradiated with the LED light at an angle, the photo diode can detect reflecting light and scattering light by the artificial bone.

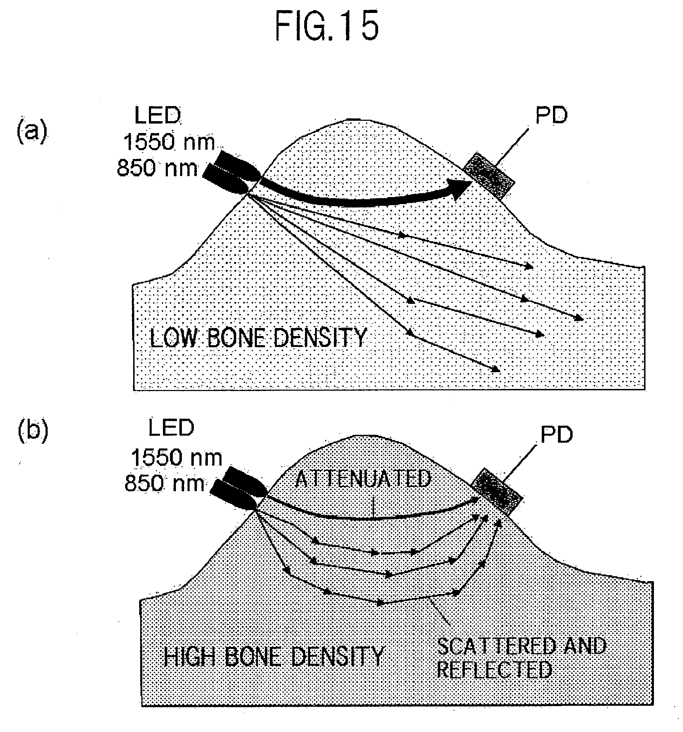

Schematic diagrams in FIG. 15 describe transmission, reflection, and dispersion of light in the working example for measuring. FIG. 15(a) shows the case of low bone density. FIG. 15(b) shows the case of high bone density. As shown in FIGS. 15(a) and 15(b), with light of wavelength 1550 nm capable of being greatly absorbed by the bone, a reduced amount of light is detected by the photo diode as the bone density increases. On the other hand, since not much light of the wavelength 850 nm is absorbed by the bone, more reflected and scattered amount of light reaches the photo diode and the calculated absorbance seems to decrease as the density increases. As a result, it is understood that change in ratio of absorbance and/or change in difference in absorbance of the two wavelengths increases as bone density changes, and thus the linear slopes indicating the relationships with the density are smaller.

According to the above finding, it can be said that when evaluating bone density from light, bone density may be predicted more sensitively by selecting near-infrared light of two wavelengths more greatly differing in change in absorbance as bone density changes, positioning the light emitter and light receiver so as to receive transmitted light and reflected and scattered light, and taking into account use of reflecting light and scattering of light in addition to the transmitted light.

<Other Embodiments>

In the above, two wavelengths are used to find correlation with bone density; however, number of wavelengths used is not limited to two. In order to also avoid influences of multiple tissues aside from bone such as skin and bone marrow, other wavelengths may be used to remove influences thereof.

For example, a measuring subject is constituted by bone tissue and skin, and the bone tissue is constituted by bone and bone marrow. Absorbance (including attenuation due to scattering) measured from a single wavelength ($\lambda_1$) may be represented by a relationship as in the following equations:

$$[\mu_a = \alpha \mu_a^{bt} + (1-\alpha) \mu_a^{s}]_{\lambda=\lambda 1} \quad \cdots\cdots (1)$$

$$[\mu_a^{bt} = \beta \mu_a^{b} + (1-\beta) \mu_a^{m}]_{\lambda=\lambda 1} \quad \cdots\cdots (2)$$

In Equation (1), $\mu_a$ denotes measured absorbance of the entire subject, $\mu_a^{bt}$ denotes absorbance of the bone tissue, $\mu_a^{s}$ denotes absorbance of the skin, and $\alpha$ denotes existence rate of the bone tissue. Similarly in Equation (2), $\mu_a^{b}$ denotes absorbance of the bone, $\mu_a^{m}$ denotes absorbance of the bone marrow, and $\beta$ denotes existence rate of the bone or desired bone density. Resulting from Equations (1) and (2),

$$[\mu_a = \alpha (\beta \mu_a^{b} + (1-\beta) \mu_a^{m}) + (1-\alpha) \mu_a^{s}]_{\lambda=\lambda 1} \quad \cdots\cdots (3)$$

It is understood from Equation (3) that the measured value $\mu_a$ is determined from the bone density ($\beta$) and existence rate of skin (1-$\alpha$). Therefore, when ratio of absorbance or difference in absorbance is measured for two wavelengths, while the relationship between that ratio and bone density ($\beta$) may be approximated to be a linear relationship as mentioned above, existence of skin influences that relationship. It is preferable to measure using more than two wavelengths in order to achieve high accuracy in measurements without any influences of skin.

From this, a method of developing a database representing relationships between measured value $\mu_a$, used wavelength, bone density, ratio of skin, and the like for each of multiple wavelengths and using it for further accurate bone density evaluation is possible. A true value prediction algorithm based on such a database includes a look-up table method, a neural network, a multivariate analysis, or the like.

Measuring with at least three wavelengths reduces influences of skin, muscles, and the like, provided that two wavelengths are the same as those used to measure the above-given bone density.

Use of a single light receiving element is possible by making multiple light emitting elements, each emitting light of a different wavelength, emit light sequentially in turn. Note that if LEDs are used as the light emitting elements, the group of those elements emitting light of multiple wavelengths is sufficiently small and can be used as a single light emitting element.

**Claims**

1. A bone density measuring device, comprising:

    a light emitter, which emits light of at least two wavelengths;
    a light receiver, which receives light from the light emitter via a bone; and
    a control unit, which is connected to the light emitter and the light receiver to control the light emitter, input a signal from the light receiver, and display it as bone density based on absorbance of light of a plurality of wavelengths.

2. The bone density measuring device of Claim 1, wherein
    the light emitter emits light of two wavelengths, and
    the control unit displays bone density based on ratio of absorbance or difference in absorbance of light of the two wavelengths.

3. The bone density measuring device of Claim 2, wherein
    the light emitter emits near-infrared light of two wavelengths $\lambda_1$ and $\lambda_2$ greater different from each other in change in absorbance as bone density changes.

4. The bone density measuring device of Claim 3, wherein
the light emitter emits light included in a wavelength region of a near-infrared LED used as the light emitter where absorption of the light included in the wavelength region by skin, water, and fat is the minimum and where the light is a combination of two wavelengths ($\lambda_1$ and $\lambda_2$) providing correlation coefficient (r) of bone density and ratio of absorbance or difference in absorbance which is 0.99 or greater and slope (s) which is 10,000 or less.

5. The bone density measuring device of any one of Claims 1 through 4, wherein
the control unit drives two light-emitting elements of the light emitter alternatively to emit light of two wavelengths; and
the light receiver is controlled so as to time-division multiplex and receive light of the two wavelengths by a single light receiving element.

6. The bone density measuring device of Claim 1, wherein
the light emitter drives a plurality of light emitting elements sequentially in turn and emits light of a plurality of wavelengths; and
the light receiver receives light of the plurality of wavelengths by a single light receiving element.

7. The bone density measuring device of any one of Claims 1 through 6, wherein
the light emitter and the light receiver are deployed so as to receive transmitted light and reflected and scattered light via the bone.

# FIG.1

# FIG.2

## FIG.3-1

$y=9142x-8867$
$r=0.851$

BONE DENSITY [mg/cm$^3$]

RATIO [A$_{\lambda 2}$/A$_{\lambda 1}$]

## FIG.3-2

$y = 9721.1x - 9439.5$
$r = 0.918$

BONE DENSITY [mg/cm$^3$]

RATIO [A$_{\lambda 2}$/A$_{\lambda 1}$]

# FIG.3-3

$y = 4553.2x + 281.69$
$r = 0.919$

BONE DENSITY [mg/cm$^3$] (y-axis: 0, 100, 200, 300, 400, 500)

DIFFERENCE [$A_{\lambda 2} - A_{\lambda 1}$] (x-axis: -0.08, -0.06, -0.04, -0.02, 0, 0.02, 0.04)

# FIG.4

( a )

( b )

1721 mg/cm³

344 mg/cm³

220 mg/cm³

1mm

# FIG.5

## ( a )

## ( b )

## ( c )

FIG.6-1

# FIG.6−2

(a)

(b)

## FIG.7

## FIG.8

# FIG.9-1

# FIG.9-2

(a)

(b)

# FIG.10

(a)

150:SKIN

180:CORTICAL BONE

130:PD

121:LED#1

122:LED#2

ULNA ROD-SHAPED PART

(b)

TARGET
AREA

# FIG.11

## （a）

110:HOLDER 115:GRIP

50mm 120mm

## （b）

121 122 130:PD

# FIG.12

## (a)

LED#1
$\lambda_1$:1200nm

450ms

0V

LED#2
$\lambda_2$:1550nm

0V

450ms

## (b)

$\lambda_1$:1200nm | $\lambda_2$:1550nm Io

$\lambda_1$:1200nm

$\lambda_2$:1550nm I

INTENSITY (V)

TIME (ms)

# FIG.13-1

(a)

850nm; 1550nm

$y = 190.08x - 636.7$
$r = 0.934$

DENSITY (mg/cm³)

RATIO

(b)

1050nm; 1550nm

$y = 285.92x - 584.13$
$r = 0.955$

DENSITY (mg/cm³)

RATIO

(c)

1200nm; 1550nm

$y = 546.17x - 964.31$
$r = 0.896$

DENSITY (mg/cm³)

RATIO

# FIG.13-2

(a)

**850 nm; 1550 nm**

DIFFERENCE
IN ABSORBANCE

RATIO OF
ABSORBANCE

$y = 426.37x - 1119.1$
$r = 0.988$

$y = 110.19x - 475.57$
$r = 0.956$

DENSITY (mg/cm³) vs RELATIVE ABSORBANCE

(b)

**1050 nm; 1550 nm**

DIFFERENCE
IN ABSORBANCE

RATIO OF
ABSORBANCE

$y = 372.39x - 783.93$
$r = 0.946$

$y = 293.09x - 872.31$
$r = 0.994$

DENSITY (mg/cm³) vs RELATIVE ABSORBANCE

(c)

**1200 nm; 1550 nm**

DIFFERENCE
IN ABSORBANCE

$y = 355.06x - 537.83$
$r = 0.968$

RATIO OF
ABSORBANCE

$y = 393.34x - 700.07$
$r = 0.934$

DENSITY (mg/cm³) vs RELATIVE ABSORBANCE

# FIG.14

# FIG.15

(a)

LED
1550 nm
850 nm

PD

LOW BONE DENSITY

(b)

LED
1550 nm
850 nm

PD

ATTENUATED

HIGH BONE DENSITY

SCATTERED AND
REFLECTED

## INTERNATIONAL SEARCH REPORT

| International application No. |
|---|
| PCT/JP2006/308432 |

| A. CLASSIFICATION OF SUBJECT MATTER |
|---|
| **_A61B10/00_**(2006.01), **_G01N21/35_**(2006.01) |

According to International Patent Classification (IPC) or to both national classification and IPC

| B. FIELDS SEARCHED |
|---|

Minimum documentation searched (classification system followed by classification symbols)
**_A61B10/00_**(2006.01), **_G01N21/35_**(2006.01)

Documentation searched other than minimum documentation to the extent that such documents are included in the fields searched
Jitsuyo Shinan Koho        1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)
JMEDPlus(JDream2)

| C. DOCUMENTS CONSIDERED TO BE RELEVANT |
|---|

| Category* | Citation of document, with indication, where appropriate, of the relevant passages | Relevant to claim No. |
|---|---|---|
| P,X | Yuya FUSHIMI et al., "Kin Sekigai Bunkoho ni yoru Kotsumitsudo Keisoku no Kisoteki Kento", Dai 44 Kai Japanese Society for Medical and Biological Engineering, 25 April, 2005 (25.04.05), Vol.43 | 1-7 |
| X | JP 2003-505130 A  (TeraView Ltd.), | 1,6,7 |
| A | 12 February, 2003 (12.02.03), Par. Nos. [0098] to [0101] | 2-5 |
| A | JP 9-70404 A  (Akira ITABASHI), 18 March, 1997 (18.03.97), Par. Nos. [0011] to [0038]; Figs. 1 to 3 | 1-7 |
| A | US 2002/0002336 A1  (Kevin S. MARCHITTO), 03 January, 2002 (03.01.02), Par. Nos. [0025] to [0085] | 1-7 |

| ☒ | Further documents are listed in the continuation of Box C. | ☒ | See patent family annex. |
|---|---|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 08 May, 2006 (08.05.06) | 16 May, 2006 (16.05.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/308432

C (Continuation).   DOCUMENTS CONSIDERED TO BE RELEVANT

| Category* | Citation of document, with indication, where appropriate, of the relevant passages | Relevant to claim No. |
|---|---|---|
| A | Akira TAKEUCHI et al., "Hikari Sakuran ni Motoduku Honesoshiki no Atarashii Sokuteiho", The Japanese Society for Bone and Mineral Research Zasshi, 01 July, 1995 (01.07.95), Vol.13, Syorokugo, page 358 | 1-7 |

Form PCT/ISA/210 (continuation of second sheet) (April 2005)

## INTERNATIONAL SEARCH REPORT
Information on patent family members

International application No.

PCT/JP2006/308432

```
JP 2003-505130 A    2003.02.12    AU 778292 B2         2004.11.25
                                  AU 6008200 A         2001.02.13
                                  EP 1202664 A1        2002.05.08
                                  GB 2352512 A         2001.01.31
                                  GB 2352512 B         2002.03.13
                                  GB 9917407 D0        1999.09.22
                                  US 2005/100866 A1    2005.05.12
                                  WO 2001/06915 A1     2001.02.01

JP 9-70404 A        1997.03.18    AU 6889696 A         1997.03.27
                                  WO 97/08994 A1       1997.03.13

US 2002/0002336 A1  2002.01.03    AU 3104201 A         2001.07.31
                                  WO 2001/52739 A1     2001.07.26
```

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CONWAY JM ; NORRIS KH ; BODWELL CE.** A new approach for the estimation of body composition: infrared interactance. *The American Journal of Clinical Nutrition,* 1984, vol. 40 (6), 1123-1130